# EUROPEAN PATENT SPECIFICATION

(11) **EP 1 733 703 B1**
(45) Date of publication and mention of the grant of the patent: **22.07.2009**
(21) Application number: 06012270.2
(22) Date of filing: 14.06.2006
(51) Int. Cl.: A61F 2/20

(54) **Voice prosthesis device**
Stimmprothesenvorrichtung
Dispositif de prothèse vocale

(30) Priority: 17.06.2005 US 691977 P; 02.06.2006 US 421898
(43) Date of publication of application: 20.12.2006
(73) Proprietor: Helix Medical, LLC, Plymouth, MI 48170 (US)
(72) Inventor: Nelson, Jesse N., Oxnard,CA 93033 (US)
(74) Representative: Pfenning, Meinig & Partner GbR

(56) References cited:
- WO-A-97/45075
- DD-A1- 275 183
- US-A1- 2004 187 941
- US-A1- 2004 193 265

## Description

### Field of the Invention

This invention relates to speech prostheses which permit the production of alaryngeal speech. It is disclosed in the context of a particular type of such a voice prosthesis. However, it is believed to be useful with other types of voice prostheses as well.

### Background of the Invention

A typical prosthesis comprises a body section which defines a passageway from one end to the other, a locating means to maintain the body in the intended place, typically between the trachea and esophagus of a laryngectomy patient, and a valve means to selectively dose the passageway through the body section. This valve means typically includes a valve seat and a movable valve element. The valve seat and the valve element form a valve element-valve seat interface when the valve element is in the "closed" position.

Various voice prostheses for residing in punctures in the tracheoesophageal walls of laryngectomees for the production of alaryngeal speech are known. There are, for example, the devices illustrated and described in the following published U.S. patent applications and issued U.S. Patents: 2005/0256573; 2005/0171602; 2004/0204759; 2004/0193265; 2004/0187941; 2002/0193879; 6,948,526; 6,776,797; 6,484,345; 5,976,151; 5,919,231; 5,693,097; 5,632,775; 5,578,083; 5,571,180; 5,507,809; 5,480,432; 5,314,470; 5,300,119; 5,064,433; 5,059,208; 5,027,812; 5,019,096; 4,911,716; 4,820,304; 4,808,183; 4,614,516; 4,610,691; 4,43 5,853: published PCT application WO 03/057082: and, European Patent 0959816.

The international application WO 97/45075 describes a further voice prosthesis to be mounted in a fistula between trachea and esophagus with a through-passage and a valve mechanism controlling the connection to said passage and having sealing surfaces which can be pressed against each other, means being provided to produce a magnet force acting between the sealing surfaces to keep said surfaces pressed against each other in a closed position of the valve mechanism. Here hard candida-resistant material is used as a layer or coating on the double flap of the valve, which may be a magnet or other hard material.

This listing is not intended to be a representation that a complete search of all relevant art has been made, or that no more pertinent art than that listed exists, or that the listed art is material to patentability. Nor should any such representation be inferred.

Efforts to increase the dwelling time of such prostheses in the tracheoesophageal wall of a wearer have been noted in several of the above identified references. The reasons for increasing the dwelling time include the desire for the convenience of not having to remove the device on a daily basis.

Voice prosthesis devices of these types are generally constructed from relatively soft, pliable biocompatible materials, for example, various low durometer silicones. In the typical voice prosthesis of the above identified types, the valve comprises a relatively thin valve element which is designed to close against a valve seat. Colony-forming microorganisms that occasionally infect mucus membranes of the throat and trachea can grow on the voice prosthesis and distort or interfere with the valve incorporated in the voice prosthesis. Such distortion may render the valve incapable of closing. Consequently, it is not uncommon for a wearer of a voice prosthesis device of these general types to have to clean the *device in situ,* or remove the device, for cleaning or for replacement.

The failure mechanisms due to microbial infestation are generally of one or more of the following four types. First, microbial colonies infest the valve seat at the movable valve member-valve seat interface, preventing the movable valve member from completely occluding the passageway through the valve seat. Second, microbial colonies infest the movable valve member at the movable valve member-valve seat interface, preventing the movable valve member from completely occluding the passageway through the valve seat. Third, microbial colonies infest the top side of the movable valve member opposite the valve seat. As colonies become established, the amount of material on this side of the movable valve member expands, deforming the opposing sealing surface of the valve seat. Finally, microbial colonies infest the inner wall of the voice prosthesis body and/or the edge of the movable valve member, preventing the movable valve member from fully closing on the valve seat.

Among the structures illustrated in the above identified references are ones in which the valve seat is constructed of a material that is stiff in relation to the material of the movable valve member. Failure of the valve due to microbial infestation is reduced somewhat, but the relatively thin movable valve member is still susceptible to infestation, increasing the risk of failure of the valve structure.

Another structure which has been explored has the movable valve member and the valve seat both made of stiffer materials. Experience with these designs has established that any small irregularity on either the movable valve member or the valve seat can lead to leakage past the valve.

### Disclosure of the Invention

According to an aspect of the invention, a voice prosthesis device provides a passageway between a tracheal end of the device and an esophageal end of the device. A valve in the passageway includes an elastomeric seat having a first hardness and a movable valve member having a second hardness harder than the first hardness. The movable valve member closes against the seat to close the valve.

Illustratively according to this aspect of the invention, the valve comprises a ring providing a seat. A hinge member is provided on the ring. The hinge member and ring are unitarily formed from elastomeric material.

Alternatively illustratively according to this aspect of the invention, the valve comprises a seat and a hinge member at the esophageal end of the device. The hinge member and seat are formed from an elastomeric material.

Alternatively illustratively according to this aspect of the invention, the valve comprises a seat in the passageway, a hinge member in the passageway, the hinge member and seat being formed from elastomeric material.

Illustratively according to these aspects of the invention, one of the movable valve member and hinge member includes a first feature and the other of the movable valve member and hinge member includes a complementary second feature for engaging the first feature to mount the movable valve member from the hinge member.

Alternatively illustratively according to these aspects of the invention, one of the movable valve member and hinge member includes a first feature and the other of the movable valve member and hinge member includes a second feature. The device further includes a third member including a first portion complementarily configured to the first feature for engaging the first feature and a second portion complementarily configured to the second feature for engaging the second feature to mount the movable valve member from the hinge member.

Alternatively illustratively according to these aspects of the invention, the device includes an adhesive for mounting the movable valve member from the hinge flap.

According to another aspect of the invention, a voice prosthesis device provides a passageway between a tracheal end of the device and an esophageal end of the device. A valve is formed at the esophageal end of the passageway. The valve includes a first member that is elastomeric and has a first hardness and a second member having a second hardness greater than the first hardness, the second member closing against the first member to close the valve.

Illustratively according to this aspect of the invention, the first member is formed from an elastomeric material and the second member is formed from a less elastic material than the elastomeric material.

Further illustratively according to this aspect of the invention, the first member and passageway are unitarily formed from the elastomeric material.

Additionally illustratively according to this aspect of the invention, the device comprises a duckbill voice prosthesis having a somewhat dome-shaped or conical esophageal end formed from an elastomeric material and provided with a slit forming the valve.

### Brief Description of the Drawings

The invention may best be understood by referring to the following description and accompanying drawings which illustrate the invention. In the drawings:
Fig. 1 illustrates a fragmentary lateral sectional view showing a wearer of a device constructed according to the invention;
Fig. 2 illustrates a perspective view, taken from the esophageal end, of the device illustrated in Fig. 1;
Fig. 3 illustrates a fragmentary end elevational view of the device illustrated in Figs. 1-2;
Fig. 4 illustrates a fragmentary longitudinal sectional view through the device illustrated in Figs. 1-3 taken generally along section lines 4, 5-4, 5 of Fig. 3, with the valve in the closed orientation;
Fig. 5 illustrates a fragmentary longitudinal sectional view through the device illustrated in Figs. 1-4 taken generally along section lines 4, 5-4, 5 of Fig. 3, with the valve in an open orientation;
Fig. 6 illustrates a perspective view of a detail of the device illustrated in Figs. 1-5;
Fig. 7 illustrates an exploded perspective view of the detail illustrated in Fig. 6;
Fig. 8 illustrates a much enlarged perspective of a longitudinal sectional view through the detail illustrated in Figs. 6-7, taken generally along section lines 8-8 of Fig. 6;
Fig. 9 illustrates an alternative construction to the construction illustrated in Fig. 8;
Fig. 10 illustrates an alternative construction to the construction illustrated in Figs. 8-9;
Fig. 11 illustrates an alternative construction to the construction illustrated in Figs. 8-10;
Fig. 12 illustrates an alternative construction to the construction illustrated in Figs. 8-11;
Fig. 13 illustrates an alternative construction to the construction illustrated in Figs. 8-12;
Fig. 14 illustrates a perspective of a longitudinal sectional view through another device constructed according to the invention;
Fig. 15 illustrates another perspective of a longitudinal sectional view through the device illustrated in Fig. 14;
Fig. 16 illustrates a much enlarged perspective of a detail of the device illustrated in Figs. 14-15;
Fig. 17 illustrates a perspective of a longitudinal sectional view through another device constructed according to the invention;
Fig. 18 illustrates a perspective of a longitudinal sectional view through another device constructed according to the invention;
Fig. 19 illustrates a perspective of a longitudinal sectional view through another device constructed according to the invention;
Fig. 20 illustrates a perspective of a longitudinal sectional view through another device constructed according to the invention;
Fig. 21 illustrates a longitudinal sectional view through another device constructed according to the invention;
Fig. 22 illustrates a longitudinal sectional view through another device constructed according to the invention;
Fig. 23 illustrates a perspective of a partial longitudinal sectional view through another device constructed according to the invention; and,
Fig. 24 illustrates a much enlarged perspective view of a detail of the device illustrated in Fig. 23.

### Detailed Descriptions of Illustrative Embodiments

The illustrated voice prosthesis devices incorporate movable valve member materials that are stiff in relation to the valve seat materials. These devices are also designed so that they can accommodate wide ranges of materials, such as antimicrobial materials, low adhesion materials, and non-silicone materials. The illustrative embodiments use typical soft silicones for all elastomeric components.

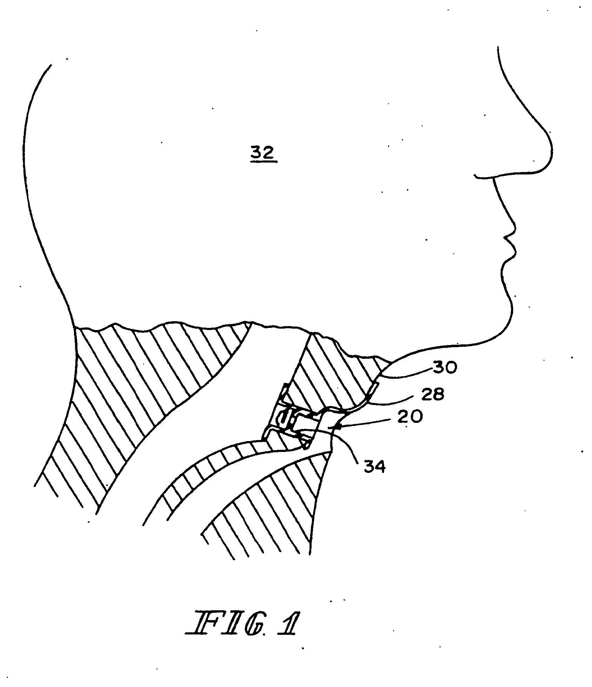

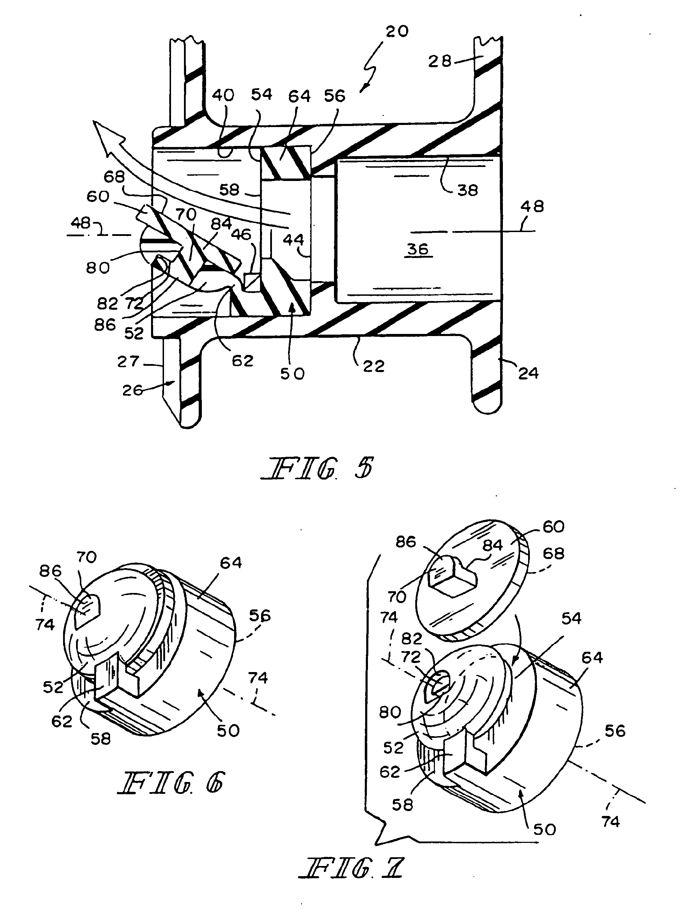

Referring to Figs. 1-5, a voice prosthesis device 20 includes a generally right circular cylindrical barrel 22 having a tracheal flange 24 at a tracheal end thereof and an esophageal flange 26 at an esophageal end thereof. While the illustrated barrel 22 is generally right circular cylindrical, it is understood that the word "cylindrical," as used in this application, is used in the mathematical sense, that is, to describe the body defined by a line moving in a closed plane path always parallel to another line. When the closed plane path is a circle, the cylinder is a circular cylinder; when an ellipse, a elliptical cylinder, and so on. When the ends of the cylinder are perpendicular to its axis, the cylinder is called a right cylinder. As best illustrated in Fig. 1, the surface 27 of esophageal flange 26 facing away from barrel 22 is contoured as, and for the reasons, described in, WO 03/057082. The esophageal flange 26 is also somewhat "teardrop" shaped in elevation to aid in orienting device 20 properly during insertion and in maintaining its orientation once inserted. The barrel 22 illustratively is molded from 50-60 durometer silicone, such as, for example, NuSil Technology med-4960 silicone.

As is customary of such devices 20, the tracheal flange 24 is provided with a strap 28 for fixing, for example, with adhesive tape, to the paratracheal skin 30 of the wearer 32. Additionally, the strap 28 may include attachment means such as a hole therethrough (not shown) for cooperating with attachment means such as a pin on an insertion tool (not shown) with the aid of which the device 20 may be inserted into a tracheoesophageal puncture 34 of the wearer 32 to aid in the production of alaryngeal speech, all in accordance with well-known principles.

Barrel 22 includes a passageway 36 which in the illustrated embodiment is also generally right circular cylindrical. The inside diameter of sidewall 38 of the passageway 36 from the tracheal end 24 is slightly larger. The inside diameter of the sidewall 40 of the passageway 36 from the esophageal end 26 is slightly smaller. At a boundary 42 between diameters 38 and 40, the inside diameter is reduced to a smaller diameter than the diameter of either sidewall 38 or sidewall 40, providing a radially inwardly extending stop 44. A number, illustratively two, of locating abutments 46 project radially inwardly toward a longitudinal axis 48 of device 20.

An elastomeric cartridge 50 including an integral, for example, molded, hinge flap 52 and a valve seat 54 is molded into barrel 22. Locating abutments 46 help orient the hinge flap 52/valve seat 54 in the barrel 22, and are also believed to aid in preventing buildup of microbial growth next to the hinge rise element. Cartridge 50 includes a tracheal end 56 and an esophageal end 58. Esophageal end 58 includes an elastomeric tab 62 by which elastomeric hinge flap 52 is attached to, and projects inwardly toward the center of, an elastomeric ring 64. Hinge flap 52/valve seat 54 can be constructed from, for example, silicone, including antimicrobial silicone, solvent-stable silicone (for example, fluorosilicone), ethylene-vinyl acetate (hereinafter sometimes EVA), polyvinyl chloride (hereinafter sometimes PVC), urethane, fluoropolymer, and so on.

Cartridge 50 further includes a hard insert 60 providing the facing surface 68 of the movable valve member 52, 60 that cooperates with the valve seat 54 to close the passageway 36 when the tracheal end 24 of the device 20 does not exceed the pressure at the esophageal end 26 by a desired amount determined by, for example, the preload on the valve. Insert 60 includes a tab 70 on its rearward side, that is, the side facing away from valve seat 54 and toward the esophageal end 26 when the valve 52, 60, 54 is closed. Hinge flap 52 includes a complementarily configured feature 72, permitting tab 70 to be snapped into feature 72 during assembly. Tab 70 and feature 72 are so configured to reduce the likelihood that insert 60 will work loose from hinge flap 52. Additionally, the elastomeric material from which hinge flap 52 is constructed elastically captures tab 70 in feature 72 further to reduce the likelihood of accidental dislodgement of insert 60 from hinge flap 52.

Insert 60 can be constructed from any suitable material including, but not limited to, filled or unfilled resins such as polyvinylidine difluoride (hereinafter sometimes PVDF), acrylonitrile-butadiene-styrene (hereinafter sometimes ABS), polyvinyl chloride (hereinafter sometimes PVC, acetal (hereinafter sometimes polyoxymethylene or POM), polytetrafluoroethylene (hereinafter sometimes PTFE), fluorinated ethylene-propylene (hereinafter sometimes FEP), perfluoroalkoxy polymer resin (hereinafter sometimes PFA), polypropylene (hereinafter sometimes PP), polyethylene (hereinafter sometimes PE), polymethyl methacrylate (hereinafter sometimes PMMA), polyamide or nylon, silicone of greater stiffness, metal, glass, ceramic, gem (ruby, sapphire, and the like), and so on.

The illustrated feature 72 includes a first, slotlike portion 80 having a first, smaller cross section and a second, larger cross section portion 82. The tab 70 includes a first, shank portion 84 having a cross section similar in shape and size to portion 80. Tab 70 also includes a second, head portion 86 having a second, larger cross section similar in shape and size to portion 82. The hinge member 52 elastically deforms to permit the passage of the head portion 86 of the tab 70 past the slotlike portion 80 of feature 72. The slotlike portion 80 of feature 72 then returns to a less deformed state, closing around shank portion 84 of tab 70. The second portion 86 of tab 70 occupies the second portion 82 of feature 72 and the first portion 84 of tab 70 occupies the first portion 80 of feature 72. Other complementary configurations of the first and second features are, of course, capable of being realized. For example, the tab could be in the form of a larger, cylindrical head on a smaller cylindrical stem, with the feature for receiving the tab being complementarily configured; or, the tab could be conical or frustoconical in shape on a smaller cross-section cylindrical stem, with the feature for receiving the tab being complementarily configured, and so on.

While the use of hard material in the construction of insert 60 may render it less resistant to microbial infestation and degradation, insert 60 may also be treated with, or incorporate, (an) antimicrobial(s) of any suitable type, such as those identified in the above noted references. However, even without such treatment, with a hard valve insert 60, the sealing surfaces of valve 52, 60, 54 are made less susceptible to the deforming effects of microbial growth simply by the stiffening and reinforcing effect of insert 60.

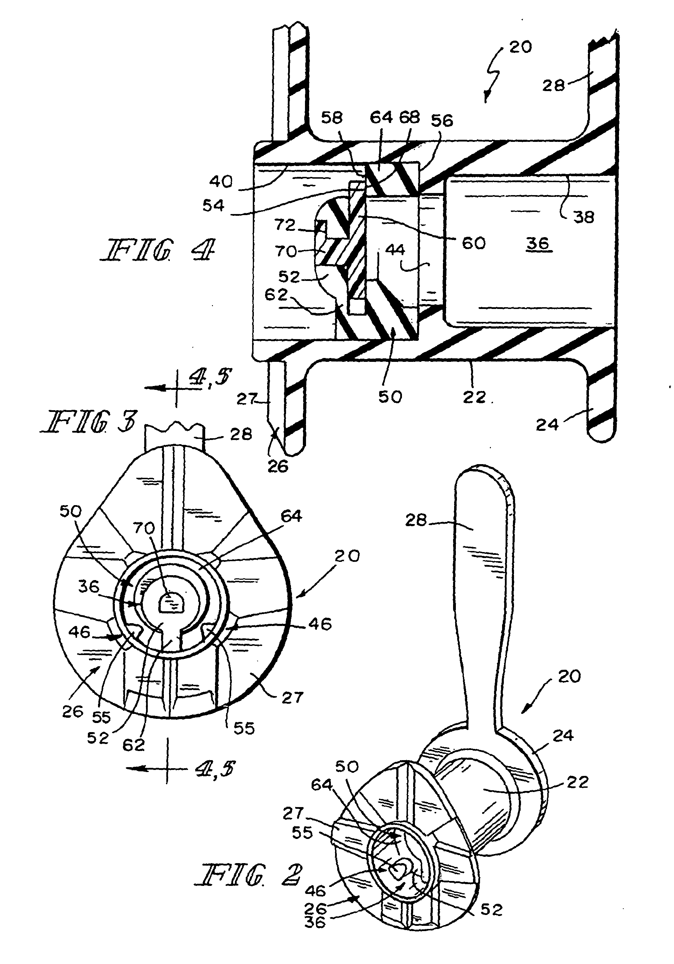

Insert 60 can be located and attached with or without glue, or can be over-molded or the like. The antimicrobial valve seat 54 and hinge flap 52 aid in increasing the useful life of the device 20. The unitary construction of valve seat 54 and hinge flap 52, and the positive location of tab 70 with respect to feature 72 increase the likelihood of positive alignment of insert 60 and valve seat 54. As may best be appreciated by referring to Figs. 3-4, the longitudinal axis 74 of the passageway 36 through cartridge 50 is offset from the axis 48 to facilitate closure of valve 52, 60, 54 without interference from tab 62.

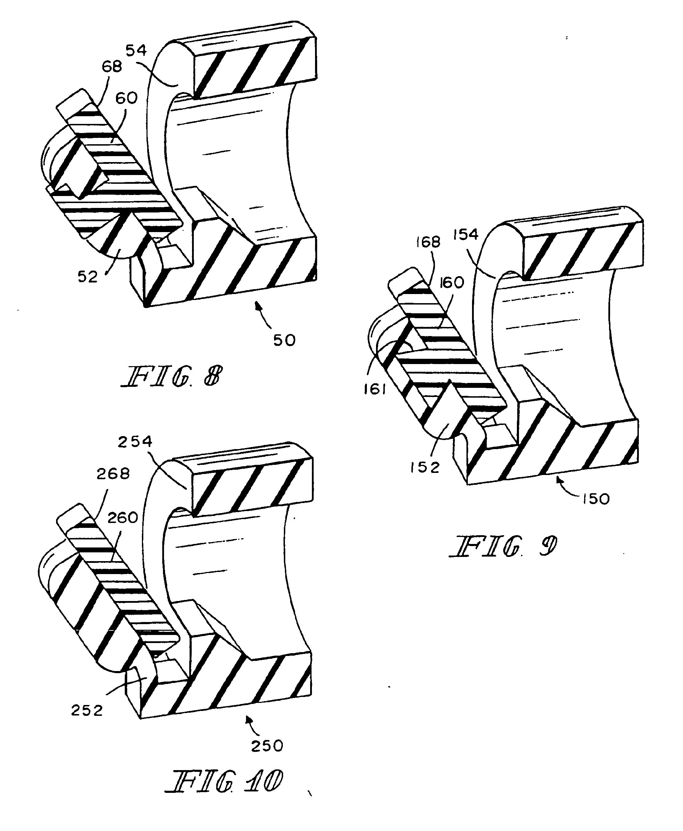

A post and lock design for coupling the material which is harder in relation to the valve seat material is illustrated in Figs. 2-8. Other techniques for coupling the material which is harder in relation to the valve seat material are illustrated in Figs. 9-16. The post and lock embodiment of Figs. 2-8 can be inverted, placed on its side, or an alternative attachment method can be used such as gluing, over molding, use of a third piece, and so on.

Fig. 9 illustrates a perspective of a longitudinal sectional view through another valve and seat assembly. An elastomeric cartridge 150 includes an integral, for example, molded, hinge flap 152 and a valve seat 154 and a hard insert 160 providing the facing surface 168 of the movable valve member 152, 160 that cooperates with the valve seat 154 to close the valve 152, 160, 154. In this embodiment, the insert 160 has a tapered stem 161 and is molded into the hinge flap 152/valve seat 154 combination when the hinge flap 152/valve seat 154 combination is molded.

Fig. 10 illustrates a perspective of a longitudinal sectional view through another valve and seat assembly. An elastomeric cartridge 250 includes an integral, for example, molded, hinge flap 252 and a valve seat 254 and a hard insert 260 providing the facing surface 268 of the movable valve member 252, 260 that cooperates with the valve seat 254 to close the valve 252, 260, 254. In this embodiment, the insert 260 is attached by a suitable adhesive to the hinge flap 252/valve seat 254 combination when the hinge flap 252/valve seat 254 combination is molded.

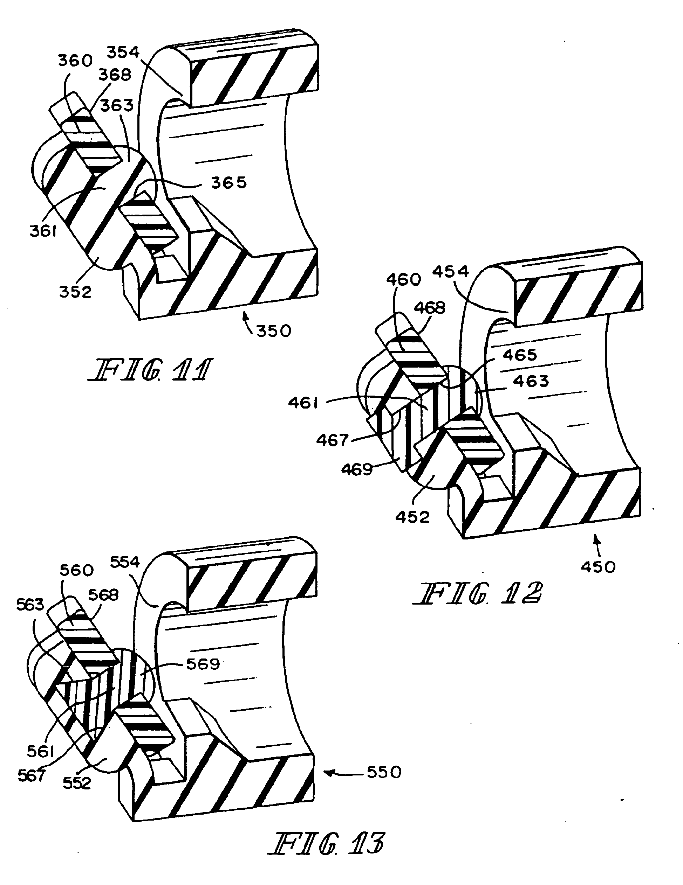

Fig. 11 illustrates a perspective of a longitudinal sectional view through another valve and seat assembly. An elastomeric cartridge 350 includes an integral, for example, molded, hinge flap 352 and a valve seat 354.and a hard insert 360 providing the facing surface 368 of the movable valve member 352, 360 that cooperates with the valve seat 354 to close the valve 352, 360, 354. In this embodiment, the hinge flap 352/valve seat 354 combination includes a somewhat mushroom-shaped stem 361 including an enlarged head 363. The hinge flap 352/valve seat 354 combination is, for example, insert molded through a passageway 365 provided for this purpose in the hard insert 360, capturing the hard insert 360 on the hinge flap 352/valve seat 354 combination.

Fig. 12 illustrates a perspective of a longitudinal sectional view through another valve and seat assembly. An elastomeric cartridge 450 includes an integral, for example, molded, hinge flap 452 and a valve seat 454 and a hard insert 460 providing the facing surface 468 of the movable valve member 452, 460 that cooperates with the valve seat 454 to close the valve 452, 460, 454. In this embodiment, the hinge flap 452/valve seat 454 combination includes a rivet 461 including an enlarged head 463. The rivet 461 is inserted through passageways 465, 467 provided for this purpose in the hard insert 460 and hinge flap 452, and the end 469 of rivet 461 is formed, capturing the hard insert 460 on the hinge flap 452/valve seat 454 combination.

Fig. 13 illustrates a perspective of a longitudinal sectional view through another valve and seat assembly. An elastomeric cartridge 550 includes an integral, for example, molded, hinge flap 552 and a valve seat 554 and a hard insert 560 providing the facing surface 568 of the movable valve member 552, 560 that cooperates with the valve seat 554 to close the valve 552, 560, 554. In this embodiment, the hinge flap 552/valve seat 554 combination includes a rivet 561 including a tapered foot 563. The rivet 561 is inserted into a cavity 567 provided for this purpose in the hinge flap 552, the hard insert 560 is placed on the stem of the rivet 561, and the end 569 of rivet 561 formed, capturing the hard insert 560 on the hinge flap 552/valve seat 554 combination.

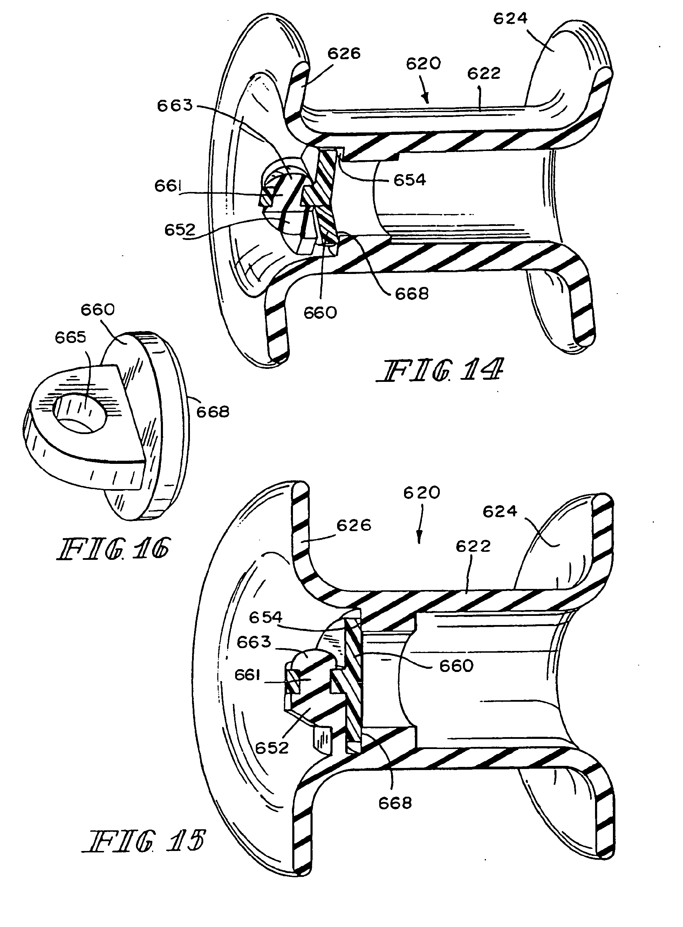

Figs. 14-16 illustrate another valve and seat assembly. An elastomeric voice prosthesis device 620 includes a generally cylindrical barrel 622 having a tracheal flange 624 at a tracheal end thereof and an esophageal flange 626 at an esophageal end thereof. As illustrated, flanges 624, 626 in this and subsequent embodiments flare outwardly gradually and continuously from the ends of barrel 622. It should be understood, however, that other known flange configurations, such as foldable flanges, teardrop-shaped flanges, and so on, could be used with the valves of these embodiments. Voice prosthesis device 620 includes an integral, for example, molded, hinge flap 652 and a valve seat 654 and a hard insert 660 providing the facing surface 668 of the movable valve member 652, 660 that cooperates with the valve seat 654 to close the valve 652, 660, 654. In this embodiment, the hinge flap 652/valve seat 654 combination includes a somewhat mushroom-shaped stem 661 including an enlarged head 663. The elastomeric voice prosthesis device 620, including hinge flap 652/valve seat 654 combination is, for example, insert molded through a passageway 665 provided for this purpose in the hard insert 660, capturing the hard insert 660 on the hinge flap 652/valve seat 654 combination.

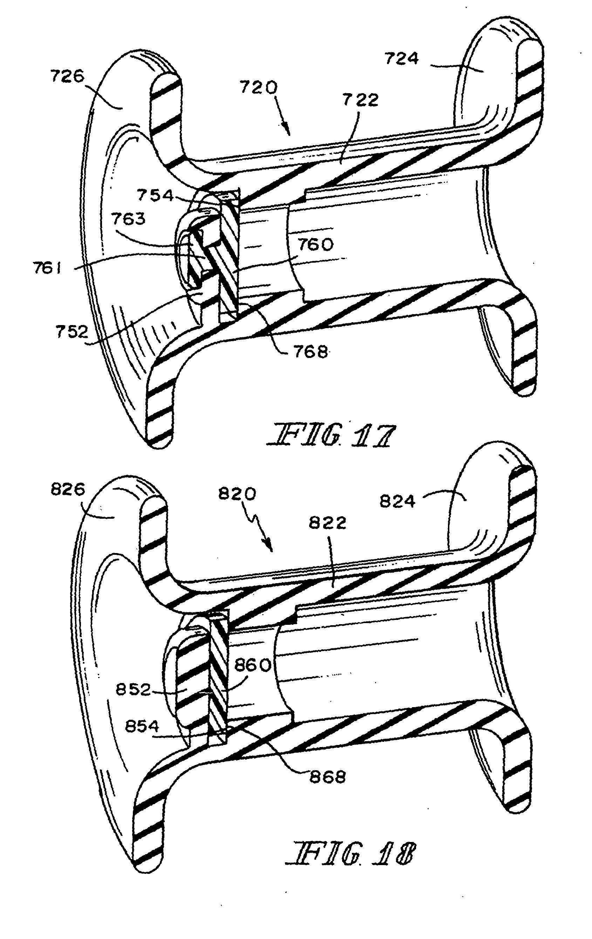

Fig. 17 illustrates another valve and seat assembly. An elastomeric voice prosthesis device 720 includes a generally cylindrical barrel 722 having a tracheal flange 724 at a tracheal end thereof and an esophageal flange 726 at an esophageal end thereof. Voice prosthesis device 720 includes an integral, for example, molded, hinge flap 752 and a valve seat 754 and a hard insert 760 providing the facing surface 768 of the movable valve member 752, 760 that cooperates with the valve seat 754 to close the valve 752, 760, 754. In this embodiment, the hinge flap 752 is insert molded around a stem 761 and head 763 of the hard insert 760 to capture the hard insert 760 on the hinge flap 752.

Fig. 18 illustrates another valve and seat assembly. An elastomeric voice prosthesis device 820 includes a generally cylindrical barrel 822 having a tracheal flange 824 at a tracheal end thereof and an esophageal flange 826 at an esophageal end thereof. Voice prosthesis device 820 includes an integral, for example, molded, hinge flap 852 and a valve seat 854 and a hard insert 860 providing the facing surface 868 of the movable valve member 852, 860 that cooperates with the valve seat 854 to close the valve 852, 860, 854. In this embodiment, the hard insert 860 is attached to the hinge flap 852 by a suitable adhesive.

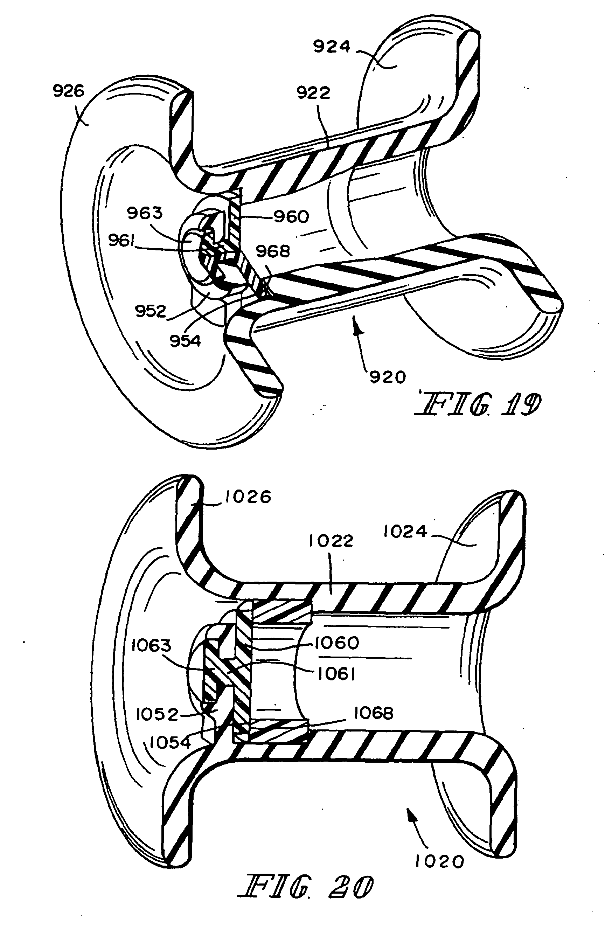

Fig. 19 illustrates a similar construction to Fig. 18, except that a valve seat 954 is separately constructed from the remainder of a barrel 922 and hinge flap 952. In this way, the valve seat 954 may be constructed from a different material from the remainder of the barrel 922 and hinge flap 952, may be made from either a harder or softer material, as the design of the voice prosthesis device 920 dictates, and may be insert molded into the remainder of the barrel 922 and hinge flap 952. Otherwise, elastomeric voice prosthesis device 920 is similar to the voice prosthesis device 820 of Fig. 18, including a tracheal flange 924 at a tracheal end thereof and an esophageal flange 926 at an esophageal end thereof. Voice prosthesis device 920 includes integral hinge flap 952 integrally molded with barrel 922 and flanges 924 and 926, and a hard insert 960 providing the facing surface 968 of the movable valve member 952, 960 that cooperates with the valve seat 954 to close the valve 952, 960, 954. In this embodiment, the hinge flap 952 is insert molded around a stem 961 and head 963 of the hard insert 960 to capture the hard insert 960 on the hinge flap 952.

Fig. 20 illustrates a similar construction to Fig. 19, with a differently configured valye seat 1054. An elastomeric voice prosthesis device 1020 includes a generally cylindrical barrel 1022 having a tracheal flange 1024 at a tracheal end thereof and an esophageal flange 1026 at an esophageal end thereof. Voice prosthesis device 1020 includes an integral, for example, molded, hinge flap 1052. Barrel 1022 and hinge flap 1052 are insert molded around valve seat 1054 and a hard insert 1060 providing the facing surface 1068 of the movable valve member 1052, 1060 that cooperates with the valve seat 1054 to close the valve 1052, 1060, 1054. The hinge flap 1052 is insert molded around a stem 1061 and head 1063 of the hard insert 1060 to capture the hard insert 1060 on the hinge flap 1052.

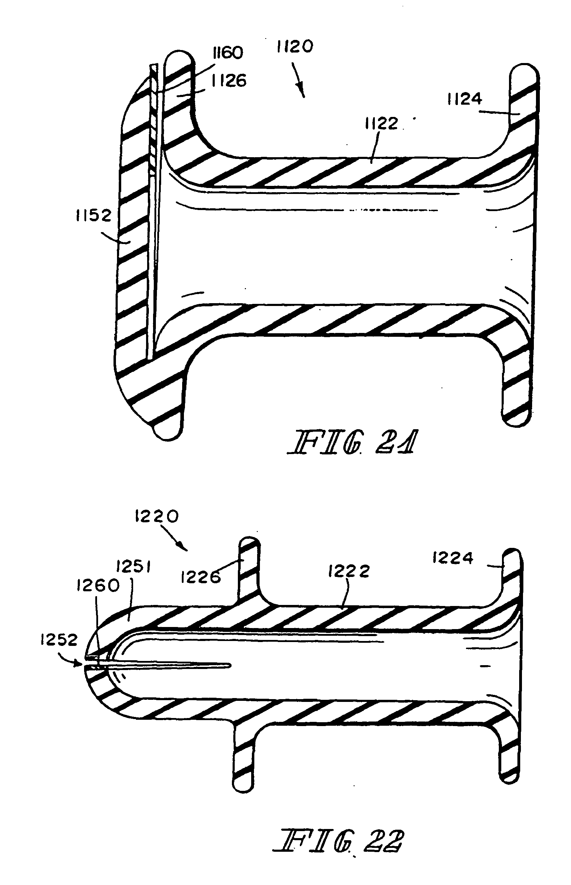

Fig. 21 illustrates another embodiment of the voice prosthesis. The voice prosthesis 1120 illustrated in Fig. 21 includes a generally cylindrical barrel 1122 having a tracheal flange 1124 at a tracheal end thereof and an esophageal flange 1126 at an esophageal end thereof. However, in the embodiment illustrated in Fig. 21, the valve includes a hinged flap 1152 integrally molded at the esophageal end thereof. The elastomeric, integrally molded barrel 1122, tracheal flange 1124, esophageal flange 1126 and hinged flap 1152 are insert molded around a hard lip insert 1160 which is constructed from a hard material.

Fig. 22 illustrates a so-called "duckbill" voice prosthesis 1220 of the general type of the Blom-Singer® duckbill prosthesis, and of the general type illustrated in Henley-Cohn et al. U.S. Patent 4,439,872; Pruitt U.S. Patent 4,596,579; and Panje U.S. Patent 4,808,183. The voice prosthesis 1220 illustrated in Fig. 22 includes a generally cylindrical barrel 1222 having a tracheal flange 1224 at a tracheal end thereof and an esophageal flange 1226 at an esophageal end thereof. A valve includes a somewhat dome-shaped or conical esophageal end 1251 integrally molded at the esophageal end of barrel 1222. The elastomeric, integrally molded nose 1251 includes at least one transversely extending slit 1252 which is illustrated in a slightly open orientation as it would appear with a slightly positive tracheal pressure with respect to the pressure in the esophagus. The barrel 1222, tracheal flange 1224 and esophageal flange 1226 are insert molded around a hard lip insert 1260 which is constructed from a hard material so that the hard lip insert 1260 forms one of the mating surfaces of the slit 1252.

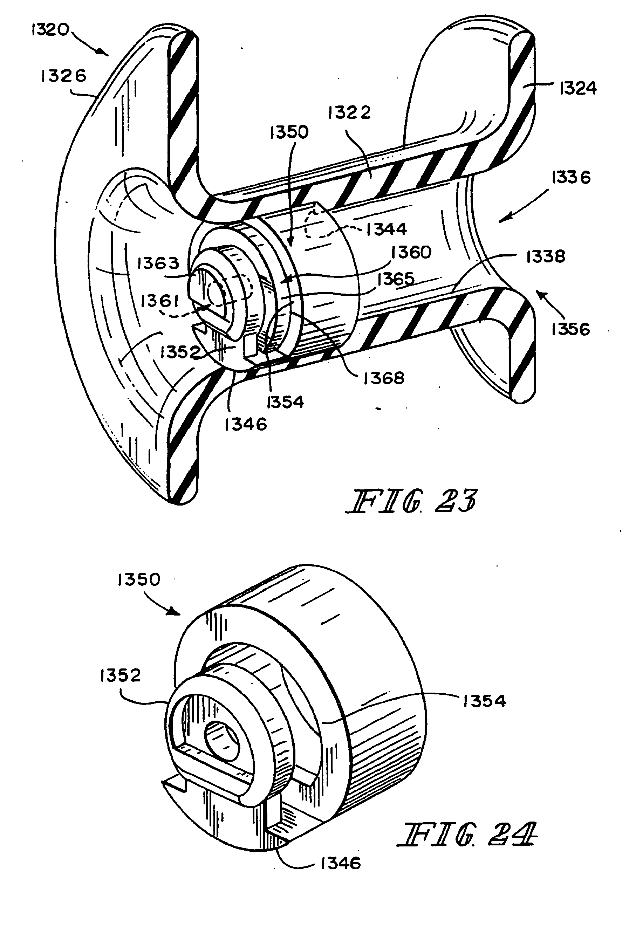

Referring to Figs. 23-24, a voice prosthesis device 1320 includes a barrel 1322 having a tracheal flange 1324 and an esophageal flange 1326. The esophageal flange 1326 is also somewhat "teardrop" shaped in elevation to aid in orienting device 1320 properly during insertion and in maintaining its orientation once inserted. The barrel 1322 illustratively is molded from 50-60 durometer silicone, such as, for example, NuSil Technology med-4960 silicone.

Barrel 1322 includes a passageway 1336, the inside diameter of sidewall 1338 of which is slightly smaller at the tracheal end, providing a radially inwardly extending stop 1344. An elastomeric cartridge 1350 includes an integral, for example, molded, hinge flap 1352 and a valve seat 1354. A combination abutment/hinge riser 1346 is believed to aid in preventing buildup of microbial growth next to the hinge riser. Again, hinge flap 1352/valve seat 1354 can be constructed from, for example, silicone, including antimicrobial silicone, solvent-stable silicone (for example, fluorosilicone), EVA, PVC, urethane, fluoropolymer, and so on.

Cartridge 1350 further includes a hard insert 1360 providing the facing surface 1368 of the movable valve member 1352, 1360 that cooperates with the valve seat 1354 to close the passageway 1336 when the pressure at the tracheal end 1324 of the device 1320 does not exceed the pressure at the esophageal end 1326 by a desired amount determined by, for example, the preload on the valve 1352, 1360, 1354. Again in this embodiment, the cartridge 1350, including hinge flap 1352, is insert molded around a stem 1361 and heads 1363, 1365 of the hard insert 1360 to capture the hard insert 1360 on the hinge flap 1352.

Again, insert 1360 can be constructed from any suitable material including, but not limited to, filled or unfilled resins such as PVDF, ABS, PVC, POM, PTFE, FEP, PFA, PP, PE, PMMA, polyamide or nylon, silicone of greater stiffness, metal, glass, ceramic, gem (ruby, sapphire, and the like), and so on.

## Claims

1. A voice prosthesis device (20) providing a passageway (36) between a tracheal end (24) of the device (20) and an esophageal end (26) of the device (20), a valve (50) in the passageway (36), the valve (50) including an elastomeric seat (54) having a first hardness and a movable valve member (60) having a second hardness harder than the first hardness, the movable valve member (60) closing against the seat (54) to close the valve (50).

2. The device of claim 1 wherein the valve (50) comprises a ring (64) providing a seat (54), a hinge member (52) provided on the ring (64), the hinge member (52) and ring (64) being unitarily formed from elastomeric material.

3. The device of claim 2 wherein one of the movable valve member (60) and hinge member (52) includes a first feature (70) and the other of the movable valve member (60) and hinge member (52) includes a complementary second feature (72) for engaging the first feature (70) to mount the movable valve member (60) from the hinge member (52).

4. The device of claim 2 wherein one of the movable valve member (460) and hinge member (452) includes a first feature (465) and the other of the movable valve member (460) and hinge member (452) includes a second feature (467), the device (20) further including a third member (461) including a first portion (463) complementarily configured to the first feature (465) for engaging the first feature (465) and a second portion (469) complementarily configured to the second feature (467) for engaging the second feature (467) to mount the movable valve member (460) from the hinge member (452).

5. The device of claim 2 including an adhesive for mounting the movable valve member (60) from the hinge member (52).

6. The device of claim 1 wherein the valve (50) comprises a seat (54), a hinge member (52) at the esophageal end (26) of the device (20), the hinge member (52) and seat (54) being formed from an elastomeric material.

7. The device of claim 6 wherein one of the movable valve member (60) and hinge member (52) includes a first feature (70) and the other of the movable valve member (60) and hinge member (52) including a complementary second (72) feature for engaging the first feature (70) to mount the movable valve member (60) from the hinge member (52).

8. The device of claim 6 wherein one of the movable valve member (460) and hinge member (452) includes a first feature (465) and the other of the movable valve member (460) and hinge member (452) includes a second feature (467), the device (20) further including a third member (461) including a first portion (463) complementarily configured to the first feature (465) for engaging the first feature (465) and a second portion (469) complementarily configured to the second feature (467) for engaging the second feature (467) to mount the movable valve member (460) from the hinge member (452).

9. The device of claim 6 including an adhesive for mounting the movable valve member (60) from the hinge member (52).

10. The device of claim 1 wherein the valve (50) comprises a seat (54) in the passageway (36), a hinge member (52) in the passageway (36), the hinge member (52) and seat (54) being formed from elastomeric material.

11. The device of claim 10 wherein one of the movable valve member (60) and hinge member (52) includes a first feature (70) and the other of the movable valve member (60) and hinge member (52) includes a complementary second feature (72) for engaging the first feature (70) to mount the movable valve member (60) from the hinge member (52).

12. The device of claim 10 wherein one of the movable valve member (460) and hinge member (452) includes a first feature (465) and the other of the movable valve member (460) and hinge member (452) includes a second feature (467), the device (20) further including a third member (461) including a first portion (463) complementarily configured to the first feature (465) for engaging the first feature (465) and a second portion (469) complementarily configured to the second feature (467) for engaging the second feature (467) to mount the movable valve member (460) from the hinge member (452).

13. The device of claim 10 including an adhesive for mounting the movable valve member (60) from the hinge member (52).

14. A voice prosthesis device (1220) providing a passageway (1222) between a tracheal end of the device (1220) and an esophageal end of the device (1220), a valve formed at the esophageal end of the passageway (1222), the valve including a first member (1251) that is elastomeric and has a first hardness and a second member (1260) having a second hardness greater than the first hardness, the second member (1260) closing against the first member (1251) to close the valve.

15. The device of claim 14 wherein the first member (1251) is formed from an elastomeric material and the second member (1260) is formed from a less elastic material than the elastomeric material.

16. The device of claim 15 wherein the first member (1251) and passageway (1222) are unitarily formed from the elastomeric material.

17. The device of claim 14 comprising a duckbill voice prosthesis (1220) having a somewhat dome-shaped or conical esophageal end (1251) formed from an elastomeric material and provided with a slit (1252) forming the valve.

18. The device of claim 1 wherein the passageway (36) and the seat (54) are unitarily formed from an elastomeric material.

19. The device of claim 18 wherein the valve (50) comprises a hinge member (52) at the esophageal end (26) of the device (20), the hinge member (52) and seat (54) being formed from an elastomeric material.

20. The device of claim 19 wherein one of the movable valve member (60) and hinge member (52) includes a first feature (70) and the other of the movable valve member (60) and hinge member (52) includes a complementary second feature (72) for engaging the first feature (70) to mount the movable valve member (60) from the hinge member (52).

21. The device of claim 19 wherein one of the movable valve member (460) and hinge member (452) includes a first feature (465) and the other of the movable valve member (460) and hinge member (452) includes a second feature (467), the device (20) further including a third member (461) including a first portion (463) complementarily configured to the first feature (465) for engaging the first feature (465) and a second portion (469) complementarily configured to the second feature (467) for engaging the second feature (467) to mount the movable valve member (460) from the hinge member (452).

22. The device of claim 19 including an adhesive for mounting the movable valve member (60) from the hinge member (52).

23. The device of claim 18 wherein the seat (54) is in the passageway (36), the valve (50) further including a hinge member (52) in the passageway (36), the hinge member (52) and seat (54) being formed from elastomeric material.

24. The device of claim 23 wherein one of the movable valve member (60) and hinge member (52) includes a first feature (70) and the other of the movable valve member (60) and hinge member (52) includes a complementary second feature (72) for engaging the first feature (70) to mount the movable valve member (60) from the hinge member (52).

25. The device of claim 23 wherein one of the movable valve member (460) and hinge member (452) includes a first feature (465) and the other of the movable valve member (460) and hinge member (452) includes a second feature (467), the device (20) further including a third member (461) including a first portion (463) complementarily configured to the first feature (465) for engaging the first feature (465) and a second portion (469) complementarily configured to the second feature (467) for engaging the second feature (467) to mount the movable valve member (60) from the hinge member (52).

26. The device of claim 23 including an adhesive for mounting the movable valve member (60) from the hinge member (52).

## Patentansprüche

1. Stimmprothesengerät (20) aufweisend einen Durchgang (36) zwischen einem Luftröhrenende (24) des Geräts (20) und einem Speiseröhrenende (26) des Gerätes, ein Ventil (50) in dem Durchgang (36), wobei das Ventil (50) einen elastomerischen Sitz (54) mit einer ersten Härte und ein bewegliches Ventilteil (60) mit einer zweiten Härte, die härter ist als die erste Härte, aufweist, wobei das bewegliche Ventilteil (60) gegen den Sitz (54) schließt, um das Ventil (50) zu schließen.

2. Gerät nach Anspruch 1, wobei das Ventil (50) einen Ring (64), der einen Sitz (54) bereitstellt, ein Gelenkteil (52), das auf dem Ring (64) angeordnet ist, aufweist, wobei das Gelenkteil (52) und der Ring (64) einheitlich aus einem elastomerischen Material geformt sind.

3. Gerät nach Anspruch 2, wobei eines von dem beweglichen Ventilteil (60) und dem Gelenkteil (52) ein erstes Merkmal (70) aufweist und das andere von beweglichem Ventilteil (60) und Gelenkteil (52) ein komplementäres zweites Merkmal (72) zum Einrasten des ersten Merkmals (70) aufweist, um das bewegliche Ventilteil (60) von dem Gelenkteil (52) zu montieren.

4. Gerät nach Anspruch 2, wobei eines von dem beweglichen Ventilteil (460) und dem Gelenkteil (452) ein erstes Merkmal (465) aufweist und das andere aus dem beweglichen Ventilteil (460) und dem Gelenkteil (452) ein zweites Merkmal (467) aufweist, wobei das Gerät (20) außerdem ein drittes Teil (461) aufweist, welches einen ersten Teil (463), der komplementär zum ersten Merkmal (465) ausgebildet ist, um mit dem ersten Merkmal (465) einzurasten und einen zweiten Teil (469), der komplementär zu dem zweiten Merkmal (467) konfiguriert ist, um mit dem zweiten Merkmal (467) einzurasten, aufweist, um das bewegliche Ventilteil (460) von dem Gelenkteil (452) zu montieren.

5. Gerät nach Anspruch 2, enthaltend einen Klebstoff zum Montieren des beweglichen Ventilteils (60) von dem Gelenkteil (52).

6. Gerät nach Anspruch 1, wobei das Ventil (50) einen Sitz (54), ein Gelenkteil (52) am Speiseröhrenende (26) des Geräts (20) aufweist, wobei das Gelenkteil (52) und Sitz (54) aus einem elastomerischen Material geformt sind.

7. Gerät nach Anspruch 6, wobei eines von beweglichem Ventilteil (60) und Gelenkteil (52) ein erstes Merkmal (70) aufweist und das andere von beweglichem Ventilteil (60) und Gelenkteil (52) ein komplementäres zweites (72) Merkmal zum Einrasten des ersten Merkmals (70) aufweist, um das bewegliche Ventilteil (60) von dem Gelenkteil (52) zu montieren.

8. Gerät nach Anspruch 6, wobei eines von beweglichem Ventilteil (460) und Gelenkteil (452) ein erstes Merkmal (465) enthält und das andere von dem beweglichen Ventilteil (460) und Gelenkteil (452) ein zweites Merkmal (467) enthält, wobei das Gerät (20) außerdem ein drittes Teil (461) enthält, welches einen ersten Teil (463), welcher komplementär zum ersten Merkmal (465) konfiguriert ist, um mit dem ersten Merkmal (465) einzurasten, und einen zweiten Teil (469), welcher komplementär zum zweiten Merkmal (467) konfiguriert ist, um mit dem zweiten Merkmal (467) einzurasten, aufweist, um das bewegliche Ventilteil (460) von dem Gelenkteil (452) zu montieren.

9. Gerät nach Anspruch 6, aufweisend einen Klebstoff zum Montieren des beweglichen Ventilteils (60) von dem Gelenkteil (52).

10. Gerät nach Anspruch 1, wobei das Ventil (50) einen Sitz (54) im Durchgang (36), ein Gelenkteil (52) im Durchgang (36) aufweist, wobei das Gelenkteil (52) und Sitz (54) aus einem elastomerischen Material geformt sind.

11. Gerät nach Anspruch 10, wobei eines von dem beweglichem Ventilteil (60) und Gelenkteil (52) ein erstes Merkmal (70) aufweist und das andere aus dem beweglichen Ventilteil (60) und dem Gelenkteil (52) ein komplementäres zweites Merkmal (72) zum Einrasten mit dem ersten Merkmal (70) aufweist, um das bewegliche Ventilteil (60) von dem Gelenkteil (52) zu montieren.

12. Gerät nach Anspruch 10, wobei eines von dem beweglichen Ventilteil (460) und Gelenkteil (452) ein erstes Merkmal (465) aufweist und das andere aus dem beweglichen Ventilteil (460) und Gelenkteil (452) ein zweites Merkmal (467) aufweist, wobei das Gerät (20) außerdem ein drittes Teil (461) aufweist, welches einen ersten Teil (463) aufweist, der komplementär zum ersten Merkmal (465) konfiguriert ist, um mit dem ersten Merkmal (465) einzurasten und einen zweiten Teil (469) aufweist, der komplementär zum zweiten Merkmal (467) konfiguriert ist, um mit dem zweiten Merkmal (567) einzurasten, um das bewegliche Ventilteil (460) von dem Gelenkteil (452) zu montieren.

13. Gerät nach Anspruch 10, aufweisend einen Klebstoff zum Montieren des beweglichen Ventilteils (60) von dem Gelenkteil (52).

14. Stimmprothesengerät (1220), welches einen Durchgang (1222) zwischen einem Luftröhrenende des Geräts (1220) und einem Speiseröhrenende des Gerätes (1220), ein am Speiseröhrenende des Durchgangs (1220) gebildetes Ventil aufweist, wobei das Ventil ein erstes Teil (1251), das elastomerisch ist und eine erste Härte hat, und ein zweites Teil (1260), das eine zweite Härte, größer als die erste Härte, hat, aufweist, wobei das zweite Teil (1260) gegen das erste Teil (1251) schließt, um das Ventil zu schließen.

15. Gerät nach Anspruch 14, wobei das erste Teil (1251) aus einem elastomerischen Material geformt ist und das zweite Teil (1260) aus einem weniger elastischen Material als das elastomerische Material geformt ist.

16. Gerät nach Anspruch 15, wobei das erste Teil (1251) und der Durchgang (1222) einheitlich aus einem elastomerischen Material geformt sind.

17. Gerät nach Anspruch 14, aufweisend eine Schnabeltierstimmprothese (1220) mit einem etwas kuppelförmigen oder konischen Speiseröhrenende (1251), das aus einem elastomerischen Material geformt ist und mit einem Schlitz (1252) ausgestattet ist, der das Ventil bildet.

18. Gerät nach Anspruch 1, wobei der Durchgang (36) und der Sitz (54) einheitlich aus einem elastomerischen Material geformt sind.

19. Gerät nach Anspruch 18, wobei das Ventil (50) ein Gelenkteil (52) am Speiseröhrenende (26) des Geräts (20) aufweist, wobei das Gelenkteil (52) und Sitz (54) aus einem elastomerischen Material geformt sind.

20. Gerät nach Anspruch 19, wobei eines von beweglichem Ventilteil (60) und Gelenkteil (52) ein erstes Merkmal (70) aufweist und das andere von beweglichem Ventilteil (60) und Gelenkteil (52) ein komplementäres zweites Merkmal (72) zum Einrasten des ersten Merkmals (70) aufweist, um das bewegliche Ventilteil (60) von dem Gelenkteil (52) zu montieren.

21. Gerät nach Anspruch 19, wobei eines von beweglichem Ventilteil (460) und Gelenkteil (452) ein erstes Merkmal (465) aufweist und das andere von beweglichem Ventilteil (460) und Gelenkteil (452) ein zweites Merkmal (467) aufweist, wobei das Gerät (20) außerdem ein drittes Teil (461) aufweist, welches einen ersten Teil (463), der komplementär zum ersten Merkmal (465) konfiguriert ist, um mit dem ersten Merkmal (465) einzurasten und einen zweiten Teil (469), der komplementär zum zweiten Merkmal (467) konfiguriert ist, um mit dem zweiten Merkmal (467) einzurasten, aufweist, um das bewegliche Ventilteil (460) vom Gelenkteil (452) zu montieren.

22. Gerät nach Anspruch 19, aufweisend einen Klebstoff zum Montieren des beweglichen Ventilteils (60) vom Gelenkteil (52).

23. Gerät nach Anspruch 18, wobei der Sitz (54) im Durchgang (36) ist, das Ventil (50) außerdem ein Gelenkteil (52) im Durchgang (63) aufweist, wobei das Gelenkteil (52) und Sitz (54) aus einem elastomerischen Material geformt sind.

24. Gerät nach Anspruch 23, wobei eines von beweglichem Ventilteil (60) und Gelenkteil (52) ein erstes Merkmal (70) aufweist, und das andere aus beweglichem Ventilteil (60) und Gelenkteil (52) ein komplementäres zweites Merkmal zum Einrasten des ersten Merkmals (70) aufweist, um das bewegliche Ventilteil (60) vom Gelenkteil (52) zu montieren.

25. Gerät nach Anspruch 23, wobei eines von dem beweglichen Ventilteil (460) und Gelenkteil (452) ein erstes Merkmal (465) aufweist und das andere von beweglichem Ventilteil (460) und Gelenkteil (452) ein zweites Merkmal (467) aufweist, wobei das Gerät (20) außerdem ein drittes Teil (461) aufweist, welches einen ersten Teil (463), der komplementär konfiguriert ist zum ersten Merkmal (465) zum Einrasten des ersten Merkmals (465), und einen zweiten Teil (469), der komplementär konfiguriert ist zum zweiten Merkmal (467) zum Einrasten des zweiten Merkmals (467), aufweist, um das bewegliche Ventilteil (60) vom Gelenkteils (52) zu montieren.

26. Gerät nach Anspruch 23, aufweisend einen Klebstoff zum Montieren des beweglichen Ventilteils (60) vom Gelenkteil (52).

## Revendications

1. Dispositif de prothèse vocale (20) fournissant une voie de passage (36) entre une extrémité trachéale (24) du dispositif et une extrémité oesophagique (26) du dispositif (20), une valve (50) dans la voie de passage (36), la valve (50) comprenant un siège en élastomère (54) ayant une première dureté (60) et un élément de valve mobile ayant une deuxième dureté plus dure que la première dureté, l'élément de valve mobile (60) se fermant contre le siège (54) pour fermer la valve (50).

2. Dispositif selon la revendication 1, dans lequel la valve (50) comprend un anneau (64) fournissant un siège (54), un élément d'articulation (52) prévu sur l'anneau (64), l'élément d'articulation (52) et l'anneau (64) étant formés d'un seul tenant à partir d'un matériau élastomère.

3. Dispositif selon la revendication 2, dans lequel un élément parmi l'élément de valve mobile (60) et l'élément d'articulation (52) comprend une première caractéristique (70) et l'autre élément parmi l'élément de valve mobile (60) et l'élément d'articulation (52) comprend une deuxième caractéristique complémentaire (72) pour mettre en prise la première caractéristique (70) pour monter l'élément de valve mobile (60) à partir de l'élément d'articulation (52).

4. Dispositif selon la revendication 2, dans lequel un élément l'un parmi l'élément de valve mobile (460) et l'élément d'articulation (452) comprend une première caractéristique (465) et l'autre élément parmi l'élément de valve mobile (460) et l'élément d'articulation (452) comprend une deuxième caractéristique (467), le dispositif (20) comprenant en outre un troisième élément (461) comprenant une première partie (463) configurée de manière complémentaire à la première caractéristique (465) pour mettre en prise la première caractéristique (465) et une deuxième partie (469) configurée de manière complémentaire à la deuxième caractéristique (467) pour mettre en prise la deuxième caractéristique (467) pour monter l'élément de valve mobile (460) à partir de l'élément d'articulation (452).

5. Dispositif selon la revendication 2, comprenant un adhésif pour le montage de l'élément de valve mobile (60) à partir de l'élément d'articulation (52).

6. Dispositif selon la revendication 1, dans lequel la valve (50) comprend un siège (54), un élément d'articulation (52) à l'extrémité oesophagique (26) du dispositif (20), l'élément d'articulation (52) et le siège (54) étant formés à partir d'un matériau élastomère.

7. Dispositif selon la revendication 6, dans lequel un élément parmi l'élément de valve mobile (60) et l'élément d'articulation (52) comprend une première caractéristique (70) et l'autre élément parmi l'élément de valve mobile (60) et l'élément d'articulation (52) comprenant une deuxième caractéristique (72) complémentaire pour mettre en prise la première caractéristique (70) pour monter l'élément de valve mobile (60) à partir de l'élément d'articulation (52).

8. Dispositif selon la revendication 6, dans lequel un élément parmi l'élément de valve mobile (460) et l'élément d'articulation (452) comprend une première caractéristique (465) et l'autre élément parmi l'élément de valve mobile (460) et l'élément d'articulation (452) comprend une deuxième caractéristique (467), le dispositif (20) comprenant en outre un troisième élément (461) comprenant une première partie (463) configurée de manière complémentaire à la première caractéristique (465) pour mettre en prise la première caractéristique (465) et une deuxième partie (469) configurée de manière complémentaire à la deuxième caractéristique (467) pour mettre en prise la deuxième caractéristique (467) pour monter l'élément de valve mobile (460) à partir de l'élément d'articulation (452).

9. Dispositif selon la revendication 6, comprenant un adhésif pour le montage de l'élément de valve mobile (60) à partir de l'élément d'articulation (52).

10. Dispositif selon la revendication 1, dans lequel la valve (50) comprend un siège (54) dans la voie de passage (36), un élément d'articulation (52) dans la voie de passage (36), l'élément d'articulation (52) et le siège (54) étant formés à partir d'un matériau élastomère.

11. Dispositif selon la revendication 10, dans lequel un élément parmi l'élément de valve mobile (60) et l'élément d'articulation (52) comprend une première caractéristique (70) et l'autre élément parmi l'élément de valve mobile (60) et l'élément d'articulation (52) comprend une deuxième caractéristique complémentaire (72) pour mettre en prise la première caractéristique (70) pour monter l'élément de valve mobile (60) à partir de l'élément d'articulation (52).

12. Dispositif selon la revendication 10, dans lequel un élément parmi l'élément de valve mobile (460) et l'élément d'articulation (452) comprend une première caractéristique (465) et l'autre élément parmi l'élément de valve mobile (460) et l'élément d'articulation (452) comprend une deuxième caractéristique (467), le dispositif (20) comprenant en outre un troisième élément (461) comprenant une première partie (463) configurée de manière complémentaire à la première caractéristique (465) pour mettre en prise la première caractéristique (465) et une deuxième partie (469) configurée de manière complémentaire à la deuxième caractéristique (467) pour mettre en prise la deuxième caractéristique (467) pour monter l'élément de valve mobile (460) à partir de l'élément d'articulation (452).

13. Dispositif selon la revendication 10, comprenant un adhésif pour le montage de l'élément de valve mobile (60) à partir de l'élément d'articulation (52).

14. Dispositif de prothèse vocale (1220) fournissant une voie de passage (1222) entre une extrémité trachéale du dispositif (1220) et une extrémité oesophagique du dispositif (1220), une valve formée à l'extrémité oesophagique de la voie de passage (1222), la valve comprenant un premier (1251) élément qui est élastomère ayant une première dureté et un deuxième élément (1260) ayant une deuxième dureté supérieure à la première dureté, le deuxième élément se fermant contre le premier élément (1251) pour fermer la valve.

15. Dispositif selon la revendication 14, dans lequel le premier élément (1251) est formé à partir d'un matériau élastomère et le deuxième élément (1260) est formé à partir d'un matériau moins élastique que le matériau élastomère.

16. Dispositif selon la revendication 15, dans lequel le premier élément (1251) et la voie de passage (1222) sont formés d'un seul tenant à partir du matériau élastomère.

17. Dispositif selon la revendication 14, comprenant une prothèse de voie vocale en bec de canard (1220) ayant une extrémité oesophagique sensiblement en forme de dôme ou conique (1251) formée à partir d'un matériau élastomère et munie d'une fente (1252) formant la valve.

18. Dispositif selon la revendication 1, dans lequel la voie de passage (36) et le siège (54) sont formés d'un seul tenant à partir d'un matériau élastomère.

19. Dispositif selon la revendication 18, dans lequel la valve (50) comprend un élément d'articulation (52) à l'extrémité oesophagique (26) du dispositif (20), l'élément d'articulation (52) et le siège (54) étant formés à partir d'un matériau élastomère.

20. Dispositif selon la revendication 19, dans lequel un élément parmi l'élément de valve mobile (60) et l'élément d'articulation (52) comprend une première caractéristique (70) et l'autre élément parmi l'élément de valve mobile (60) et l'élément d'articulation (52) comprend une deuxième caractéristique complémentaire (72) pour mettre en prise la première caractéristique (70) pour monter l'élément de valve mobile (60) à partir de l'élément d'articulation (52).

21. Dispositif selon la revendication 19, dans lequel un élément parmi l'élément de valve mobile (460) et l'élément d'articulation (452) comprend une première caractéristique (465) et l'autre élément parmi l'élément de valve mobile (460) et l'élément d'articulation (452) comprend une deuxième caractéristique (467), le dispositif (20) comprenant en outre un troisième élément (461) comprenant une première partie (463) configurée de manière complémentaire à la première caractéristique (465) pour mettre en prise la première caractéristique (465) et une deuxième partie (469) configurée de manière complémentaire à la deuxième caractéristique (467) pour mettre en prise la deuxième caractéristique (467) pour monter l'élément de valve mobile (460) à partir de l'élément d'articulation (452).

22. Dispositif selon la revendication 19, comprenant un adhésif pour le montage de l'élément de valve mobile (60) à partir de l'élément d'articulation (52).

23. Dispositif selon la revendication 18, dans lequel le siège (54) est dans la voie de passage (36), la valve (50) comprenant en outre un élément d'articulation (52) dans la voie de passage (36), l'élément d'articulation (52) et le siège (54) étant formés à partir d'un matériau élastomère.

24. Dispositif selon la revendication 23, dans lequel un élément parmi l'élément de valve mobile (60) et l'élément d'articulation (52) comprend une première caractéristique (70) et l'autre élément parmi l'élément de valve mobile (60) et l'élément d'articulation (52) comprend une deuxième caractéristique complémentaire (72) pour mettre en prise la première caractéristique (70) pour monter l'élément de valve mobile (60) à partir de l'élément d'articulation (52).

25. Dispositif selon la revendication 23, dans lequel un élément parmi l'élément de valve mobile (460) et l'élément d'articulation (452) comprend une première caractéristique (465) et l'autre élément parmi l'élément de valve mobile (460) et l'élément d'articulation (452) comprend une deuxième caractéristique (467), le dispositif (20) comprenant en outre un troisième (461) élément comprenant une première partie (463) configurée de manière complémentaire à la première caractéristique (465) pour mettre en prise la première caractéristique (465) et une deuxième partie (469) configurée de manière complémentaire à la deuxième caractéristique (467) pour mettre en prise la deuxième caractéristique (467) pour monter l'élément de valve mobile (60) à partir de l'élément d'articulation (52).

26. Dispositif selon la revendication 23, comprenant un adhésif pour le montage de l'élément de valve mobile (60) à partir de l'élément d'articulation (52).
